(19) Europäisches Patentamt
European Patent Office
Office européen des brevets

(11) Numéro de publication: **0 030 195**
**A1**

(12) # DEMANDE DE BREVET EUROPEEN

(21) Numéro de dépôt: **80401711.9**

(22) Date de dépôt: **01.12.80**

(51) Int. Cl.$^3$: **C 12 Q 1/00**
C 12 Q 1/32, C 12 Q 1/54
G 01 N 27/30

(30) Priorité: **03.12.79 FR 7929826**

(43) Date de publica…on de la demande:
**10.06.81 Bu… 81/23**

(84) Etats Contrac…ts Désignés:
**AT BE CH DE GB IT LI LU NL SE**

(71) Demandeur: **SOCIETE D'ETUDES DE TECHNIQUES ET DE REALISATIONS INDUSTRIELLES ET COMMERCIALES (SETRIC)**
**Zone Industrielle de Montaudran Boîte Postale 4050**
**Avenue Didier Daurat F-31027 Toulouse Cédex(FR)**

(72) Inventeur: **Mahenc, Jean**
**Lotissement 137 Labastide-Falgarde**
**F-31120 Lacroix Falgarde(FR)**

(72) Inventeur: **Comtat, Maurice**
**Résidence du Midi Impasse Jean Delfour**
**F-31400 Toulouse(FR)**

(72) Inventeur: **Durliat, Hélène**
**1, Résidence des Deux Ormeaux**
**F-31500 Ramonville Saint Agne(FR)**

(74) Mandataire: **Lerner, François**
**5, rue Jules Lefebvre**
**F-75009 Paris(FR)**

(54) **Procédé de mesure de la concentration d'un substrat oxydable ou réductible en solution au moyen d'une enzyme oxydo-réductase et dispositif destiné à mettre en oeuvre un tel procédé.**

(57) L'invention a trait aux réactions enzymatiques d'oxydo-réduction et elle concerne plus particulièrement un procédé utilisant ces réactions enzymatiques pour mesurer la concentration d'un substrat oxydable ou réductible en solution.

Suivant l'invention, ce procédé de mesure consiste :

– à amener le substrat de la solution à diffuser à travers une membrane semi-perméable (2) vers une chambre réactionnelle, (1b),

– à disposer une enzyme oxydo-réductase spécifique dudit substrat dans la chambre réactionnelle pour réaliser une réaction d'oxyde-réduction enzymatique entre ledit substrat et ladite enzyme dans ladite chambre,

– à régénérer la forme oxydée ou réduite de l'enzyme par une réaction électrochimique réalisée dans la chambre réactionnelle sur une électrode – anode ou cathode – dite de régénération enzymatique (4) située dans la chambre et portée à un potentiel déterminé,

– à réaliser une réaction électrochimique associée à l'extérieur de la chambre sur une électrode auxiliaire (6)-cathode ou anode- disposée dans la solution à l'extérieur de ladite chambre réactionnelle,

– et à mesurer l'intensité du courant engendré entre l'électrode de régénération enzymatique et l'électrode-auxiliaire.

L'invention concerne également un dispositif destiné à mettre en oeuvre ce procédé.

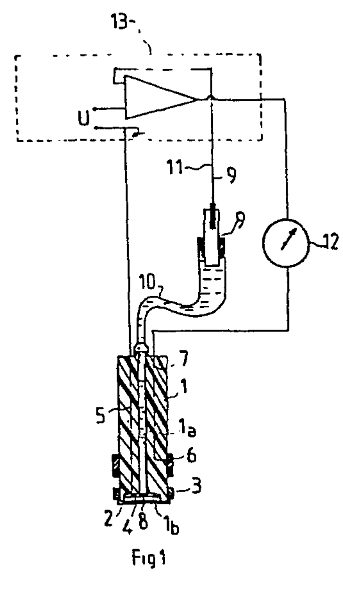

Fig1

EP 0 030 195 A1

Croydon Printing Company Ltd.

# PROCEDE DE MESURE DE LA CONCENTRATION D'UN SUBSTRAT OXYDABLE OU REDUCTIBLE EN SOLUTION AU MOYEN D'UNE ENZYME OXYDO-REDUCTASE ET DISPOSITIF DESTINE A METTRE EN OEUVRE UN TEL PROCEDE.

L'invention a trait aux réactions enzymatiques d'oxydo-réduction et elle concerne plus particulièrement un procédé utilisant ces réactions enzymatiques pour mesurer la concentration d'un substrat oxydable ou réductible en solution.

Les réactions enzymatiques d'oxydo-réduction actuellement connues consistent à utiliser une enzyme oxydo-réductase pour catalyser une réaction d'oxydation ou de réduction entre un substrat à transformer et un couple oxydo-réducteur convenablement choisi (généralement désigné par cofacteur). Dans toutes les réactions connues, l'enzyme agit comme simple catalyseur et se retrouve dans son état initial après la réaction.

Par exemple, la réaction enzymatique de transformation de l'ion lactate en ion pyruvate utilise de façon bien connue l'hexacyanoferrate III comme cofacteur et la réaction se déroule selon le mécanisme suivant :

$$CH_3\ CHOHCOO^- + 2\ Fe\ (CN)_6^{3-} \longrightarrow CH_3\ COCOO^- + 2\ Fe\ (CN)_6^{4-} + 2H^+$$

en présence de lacticodeshydrogénase.

L'hexacyanoferrate III peut être regénéré par une oxydation électrochimique sur une anode :

$$Fe\ (CN_6)^{4-} \longrightarrow Fe\ (CN_6)^{3-} + e-$$

Comme le démontre ce mécanisme classique, dans ces réactions où l'enzyme ne remplit qu'une fonction de catalyseur, le cofacteur constitue le corps de base essentiel à partir duquel s'effectue la réaction d'oxydo-réduction.

Dans de nombreuses applications, la présence de ce cofacteur est une servitude gênante. D'une part, il constitue un corps supplémentaire, onéreux dans certains cas, qu'il est nécessaire d'ajouter préalablement au substrat à traiter en même temps que

l'enzyme, et ce dans des proportions souvent précises. De plus, dans de nombreuses applications, cette addition du cofacteur au substrat conduit à contaminer ce dernier et, en conséquence, les réactions doivent être opérées sur des échantillons préalablement prélévés. Ainsi, dans l'exemple précédemmment cité, une mesure de concentration de l'ion lactate dans le sang ne peut pas être réalisée in vivo en raison des risques de contamination du sang par l'hexacyanoferrate.

De nombreuses recherches entreprises ces dernières années ont permis de réaliser une réaction enzymatique d'oxydo-réduction permettant notamment d'obtenir un substrat oxydable ou réductible sous une forme oxydée ou réduite sans la présence d'un cofacteur. A cet effet, d'une part le substrat est mis en présence avec une enzyme oxydo-réductase spécifique dudit substrat sous la forme oxydée ou réduite pour réaliser une réaction d'oxydo-réduction enzymatique dans laquelle l'enzyme joue le rôle de réactif oxydant ou réducteur et se transforme en enzyme sous forme réduite ou oxydée, d'autre part la forme oxydée ou réduite de l'enzyme est régénérée par une réaction électrochimique sur une électrode -anode ou cathode- dite de régénération enzymatique, portée à un potentiel déterminé, et enfin, une réduction ou une oxydation simultanée d'au moins un constituant de la solution de substrat est réalisée par une réaction électrochimique associée, sur une électrode auxiliaire - cathode ou anode . Les enzyme oxydoréductases agissent donc directement en tant que couple oxydo-réducteur sur une électrode conductrice et sont régénérées dans leur forme initiale par réaction électrochimique. Ces deux avantages favorisent ainsi une transformation continue du substrat en l'absence totale de cofacteur.

Pour donner un exemple représentatif de ce type de réaction enzymatique, les réactions qui se développent au cours de la transformation du lactate sans la présence du cofacteur, ont été symbolisées ci-dessous :

1°) - Oxydation par l'enzyme :

$CH_3CHOHCOO^-$ + Lactico-deshydrogénase sous forme oxydée ⟶ $CH_3COCOO^-$ + Lactico-deshydrogénase sous forme réduite + 2 $H^+$

2°) - Régénération électrochimique de l'enzyme sur anode :

Lacticodeshydrogénase réduite ⟶ Lacticodeshydrogénase oxydée + e-

3°) - Réduction électrochimique simultanée à la cathode soit de l'oxygène dissous ($1/2\ O_2 + H_2O + 2e^- \quad 2OH^-$), soit de l'eau ($2H_2O + 2e^- \longrightarrow H_2 + 2OH^-$), soit de tout autre composé réductible présent dans la solution.

Faisant le bilan de cet état de fait, les inventeurs ont mené des recherches qui ont abouti à un procédé permettant de mesurer la concentration d'un substrat en solution au moyen d'une réaction enzymatique d'oxydo-réduction et sans faire intervenir de cofacteur.

Suivant l'invention, le procédé de mesure consiste :

- à amener le substrat de la solution à diffuser à travers une membrane semi-perméable vers une chambre réactionnelle,

- à disposer l'enzyme oxydo-réductase dans la chambre réactionnelle pour réaliser la réaction d'oxydo-réduction enzymatique entre ledit substrat et ladite enzyme dans ladite chambre,

- à régénérer l'enzyme par réaction électro-chimique dans la chambre réactionnelle sur une électrode de régénération enzymatique située dans la chambre et portée à un potentiel déterminé,

- à réaliser la réaction électrochimique associée, à l'extérieur de la chambre sur une électrode auxiliaire disposée dans la solution à l'extérieur de ladite chambre réactionnelle,

- et à mesurer l'intensité du courant engendré entre l'électrode de régénération enzymatique et l'électrode auxiliaire.

Les expérimentations menées par les inventeurs ont montré que la réaction enzymatique d'oxydo-réduction et la réaction électrochimique de régénération présentent des vitesses élevées par rapport à la vitesse de diffusion du substrat à travers la membrane, de sorte que le courant mesuré est directement proportionnel à cette vitesse de diffusion et donc, à la concentration du substrat. On réalise ainsi, en l'absence de tout cofacteur, un dosage du substrat par la simple lecture d'une intensité de courant directement proportionnelle à la concentration et ce, dans des conditions opératoires très simplifiées grâce à la suppression du cofacteur.

Pour assurer une régénération électrochimique efficace de l'enzyme, l'électrode de régénération enzymatique est de préférence portée à un potentiel prédéterminé, dit potentiel de régénération, correspondant aux conditions optimales de cette régénération.

Dans chaque application, ce potentiel peut être préalablement défini en réalisant une réaction électrochimique de l'enzyme seule, en faisant varier le potentiel de l'électrode, en mesurant la vitesse de transformation de l'enzyme et en détectant le potentiel pour lequel cette vitesse présente une valeur maximale ou proche de la maximale.

Cette vitesse maximale de transformation peut facilement être détectée par une mesure de l'intensité du courant produit dans la réaction électrochimique de l'enzyme seule, cette intensité étant proportionnelle à la vitesse de transformation.

Selon un mode de mise en oeuvre préféré, une électrode de référence est disposée au voisinage de la susdite chambre réactionnelle pour permettre de fixer le potentiel de l'électrode de régénération enzymatique à une valeur sensiblement constante appropriée. Cette disposition qui utilise une référence de potentiel bien définie, permet dans chaque cas de se placer au potentiel de régénération de façon permanente et précise.

Par exemple, dans le cas où le substrat à transformer est du lactate, l'enzyme spécifique utilisée est une lacticodeshydrogénase sous forme oxydée et la différence de potentiel entre l'anode et l'électrode de référence peut être sensiblement fixée à une valeur comprise entre 450 et 550 millivolts en supposant l'électrode de référence au calomel saturé. Rappelons que l'électrode au calomel saturé présente un potentiel de + 245 millivolts par rapport à l'électrode normale à hydrogène qui par convention est la référence 0 des potentiels.

Dans le cas où le substrat à transformer est du glucose, l'enzyme spécifique utilisée est une glucose oxydase sous forme oxydée et la différence de potentiel entre l'anode et l'électrode de référence supposée au calomel saturé est sensiblement fixée à une valeur comprise entre 400 et 550 millivolts.

Dans la plupart des cas, l'enzyme sera préalablement adsorbée sur l'électrode de régénération avant l'immersion de la chambre réactionnelle dans la solution de substrat; cette disposition conduit à des manipulations très simplifiées puisqu'il n'y a plus aucun corps à mélanger à la solution.

Dans la quasi-totalité des applications, le substrat possède une masse moléculaire très inférieure à celle de son enzyme spécifique ; pour éviter une diffusion de l'enzyme à travers la membrane, diffusion qui provoquerait des pertes en

enzymes, on utilise avantageusement une membrane semi-perméable ayant une porosité définie par un seuil de coupure de valeur intermédiaire entre la masse moléculaire du substrat et celle de l'enzyme.

Cette porosité peut, dans la majorité des cas, être définie par un seuil de coupure de l'ordre de 4 000 à 6 000 ; en effet, les enzymes ont des masses moléculaires supérieures à 20 000 (lacticodeshydrogénase : 59 000 ; glucose oxydase : 100 000) et les substrats à doser sont en général de petites molécules de masse inférieure à 500.

La concentration de l'enzyme spécifique dans la chambre réactionnelle est, en général, choisie à une valeur supérieure à celle des concentrations nécessaires dans les procédés à co-facteurs, de façon que la réaction enzymatique ne soit pas limitée par un manque momentané d'enzyme.

Dans le cas du lactate ou du glucose, la concentration de l'enzyme peut approximativement être comprise entre 80 et 500 micromoles par litre.

L'invention concerne également un dispositif de mesure de la concentration d'un substrat oxydable en solution, permettant de mettre en oeuvre les concepts fondamentaux du procédé évoqué ci-dessus. Ce dispositif comprend un corps délimitant une chambre réactionnelle, une membrane semi-perméable fixée sur ce corps et fermant ladite chambre, une anode située dans cette chambre, une cathode à l'extérieur de celle-ci et une solution enzymatique contenue dans la chambre ; selon la présente invention, cette chambre contient en solution une enzyme spécifique du substrat à l'exclusion de tout cofacteur actif. De préférence l'enzyme se trouve adsorbée sur l'anode.

La description qui suit présente, en référence aux dessins annexés, un mode de réalisation d'un dispositif de mesure conforme à l'invention et des exemples 1 et 2 de mise en oeuvre pour une mesure de concentration .

Sur ces dessins :

- la figure 1 est un schéma d'ensemble du dispositif de mesure ci-dessus évoqué,
- la figure 2 est un diagramme d'étalonnage obtenu expérimentalement dans le cas du dosage du lactate,
- la figure 3 est un diagramme similaire dans le cas du dosage du glucose.

Le dispositif de mesure représenté à titre d'exemple à la figure 1 comprend un corps d'électrode 1 de forme cylindrique en matière synthétique isolante, de quelques millimètres de diamètre et de quelques centimètres de hauteur.

Ce corps est percé d'un canal central 1a et est pourvu en bout d'une partie creuse 1b de forme circulaire appelée à former une chambre réactionnelle.

Cette chambre est fermée par une membrane semi-perméable 2, en l'exemple en cellophane, qui est maintenue par une bague 3 engagée en force autour du corps 1. Le seuil de coupure de cette membrane est de l'ordre de 4000 à 6000.

Dans la partie creuse 1b est collée une pastille conductrice 4 en forme de couronne, appelée à jouer le rôle d'anode de régénération enzymatique ; cette anode est reliée à un conducteur électrique 5 traversant le corps.

Cette anode 5 dite de régénération enzymatique est de préférence réalisée en un métal ou alliage non oxydable au potentiel de travail. On évite ainsi, d'une part la contamination de la solution par des ions métalliques, d'autre part une altération de cette électrode, enfin la production de courants parasites susceptibles d'être gênants dans certaines applications.

Elle peut notamment être choisie en un métal du groupe suivant : platine, or, rhodium, irridium ou en un alliage desdits métaux.

Il est également possible de réaliser ladite anode en carbone vitreux, ou encore en un métal recouvert d'une couche d'oxyde conducteur électronique, corps qui fournissent les mêmes avantages que les métaux ci-dessus.

Dans la chambre réactionnelle est disposée une solution aqueuse tamponnée contenant l'enzyme spécifique du substrat à traiter. Cette enzyme se trouve en partie en solution et en partie naturellement adsorbée sur l'anode 4.

De plus autour du corps 1 et au voisinage de son extrémité est collé un manchon métallique 6 appelé à jouer le rôle de cathode auxiliaire et relié à un conducteur électrique 7. Cette électrode 6 sur laquelle se déroule la réaction électrochimique associée, est de préférence une électrode insoluble dans la solution de substrat et inattaquable, pour des raisons analogues à celles déjà développées pour l'anode 5 de régénération enzymatique.

Le canal 1a du corps 1 est obturé au niveau de la chambre réactionnelle 1b par un gel ou une pastille poreuse 8 et est rempli d'un électrolyte (solution aqueuse tamponnée de nature identique à celle de la chambre réactionnelle) réalisant une jonction électrolytique avec une électrode de référence de type classique 9. Un conduit souple 10 relie l'extrémité supérieure du canal à l'électrode de référence ; la liaison électrique entre cette dernière et le circuit électrique est assurée par un conducteur 11.

De façon connue en soi, l'anode, l'électrode de référence et la cathode (cette dernière par l'entremise d'un galvanomètre 12) sont connectées à un potentiostat 13 fermant les circuits et appliquant à l'anode un potentiel constant par rapport à l'électrode de référence.

Les exemples 1 et 2 qui suivent ont été conduits au moyen du dispositif ci-dessus décrit.

EXEMPLE 1

Les caractéristiques du dispositif de mesure utilisées dans cet exemple, sont les suivantes :

- anode en platine de surface de l'ordre de 6 $mm^2$,
- cathode en platine de surface de l'ordre de 20 $mm^2$,
- électrode de référence au calomel saturé, prolongée par une jonction électrolytique constituée par une solution tamponnée de $KH_2PO_4$ et $Na_2HPO_4$ de pH égal à 7,2,
- chambre réactionnelle de profondeur sensiblement égale à 80 micromètres,
- enzyme dans la chambre réactionnelle : environ 0, 2 microlitre de solution aqueuse tamponnée de lacticodeshydrogénase extraite de levure de boulangerie (souche Hansenula Anomala) de concentration 100 micromolaire.

Aucun autre corps n'est ajouté à la solution d'enzyme contenue dans la chambre réactionnelle.

Cette expérimentation a été menée dans les conditions opératoires suivantes :

- température : 21°C,
- potentiel de l'anode : + 450 millivolts par rapport à l'électrode de référence.

La solution analysée était une solution tamponné de pH 7,2 constituée par un mélange équimoléculaire de $KH_2PO_4$ et $Na_2HPO_4$, de concentration 0,2 molaire en chacun des deux constituants. A

cette solution de base on a ajouté de l'acide lactique de façon à réaliser des solutions de concentrations connues en lactate, comprises entre 0,05 et 7 millimolaire. La mesure préalable de ces concentrations a été réalisée par spectrophotométrie.

L'électrode enzymatique est à chaque mesure immergée dans la solution ; l'indication du galvanomètre se stabilise au bout d'une minute environ et la lecture est alors effectuée.

Le diagramme de la figure 2 donne les intensités I mesurées à l'état stationnaire en fonction de la concentration en lactate $C_L$. La courbe obtenue est parfaitement linéaire et permet d'étalonner directement le galvanomètre en concentration.

Des mesures de concentration effectuées après un tel étalonnage donnent un résultat avec une erreur relative de 2% environ. Le nombre de mesures effectuées a été supérieur à 100 sans que l'on constate une dérive de la réponse.

Un tel dispositif de mesure où l'enzyme lacticodeshydrogénase agit directement comme oxydant et se régénère électrochimiquement sur l'anode possède donc les mêmes performances qu'un dispositif classique à cofacteur sans présenter les inconvenients qu'amène ce dernier corps.

Dans le cas du dosage du lactate dans le sang, la mesure peut être effectuée directement in vivo en implantant le dispositif dans une veine, sans aucun risque de contamination du sang et avec une remarquable simplicité de manipulations.

EXEMPLE 2

Les caractéristiques physiques du dispositif de mesure sont les mêmes que dans l'exemple 1.

La chambre réactionnelle contient environ 0,2 microlitre de glucose oxydase (type "Sigma" grade II) de concentation 100 micromolaire ; en outre du Na $N_3$ est ajouté à titre de catalyseur de la réaction d'oxydation enzymatique à une concentration de l'ordre de 20 millimolaire. Aucun autre corps n'est ajouté dans la chambre réactionnelle.

Le potentiel de l'anode est dans ce cas porté à + 500 millivolts par rapport à l'électrode de référence.

La solution analysée était formée par une solution de base de même type que dans l'exemple précédent à laquelle on a ajouté du glucose pour réaliser des solutions de concentrations connues comprises entre 0,05 et 6 millimolaire. La mesure préalable de ces concentrations a été réalisée par pesée.

Les résultats sont analogues à ceux de l'exemple précédent et le diagramme de la figure 3 qui donne les intensités I mesurées à l'état stationnaire en fonction de la concentration en glucose $C_G$ montre que la courbe obtenue est linéaire. Aucune dérive de la réponse n'a été constatée.

R E V E N D I C A T I O N S

1. Procédé permettant de mesurer la concentration d'un substrat oxydable ou réductible en solution dans lequel d'une part, le substrat est mis en présence avec une enzyme oxydo-réductase spécifique dudit substrat sous la forme oxydée ou réduite pour réaliser une réaction d'oxydo-réduction enzymatique dans laquelle l'enzyme joue le rôle de réactif oxydant ou réducteur et se transforme en enzyme sous forme réduite ou oxydée, d'autre part la forme oxydée ou réduite de l'enzyme est régénérée par une réaction électrochimique sur une électrode - anode ou cathode- dite de régénération enzymatique, portée à un potentiel déterminé, et enfin, une réduction ou une oxydation simultanée d'au moins un constituant de la solution de substrat est réalisée par une réaction électrochimique associée, sur une électrode auxiliaire cathode ou anode -, ledit procédé étant CARACTERISE EN CE QU'il consiste :

- à amener le substrat de la solution à diffuser à travers une membrane semi-perméable vers une chambre réactionnelle,

- à disposer l'enzyme oxydo-réductase dans la chambre réactionnelle pour réaliser la réaction d'oxydo-réduction enzymatique entre ledit substrat et ladite enzyme dans ladite chambre,

- à régénérer l'enzyme par réaction électrochimique dans la chambre réactionnelle sur ladite électrode de régénération enzymatique située dans la chambre et portée à un potentiel déterminé,

- à réaliser la réaction électrochimique associée, à l'extérieur de la chambre sur ladite électrode auxiliaire disposée dans la solution à l'extérieur de ladite chambre réactionnelle,

- et à mesurer l'intensité du courant engendré entre l'électrode de régénération enzymatique et l'électrode auxiliaire.

2. Procédé de mesure selon la revendication 1, CARACTERISE EN CE QUE l'électrode de régénération enzymatique est portée à un potentiel dit de régénération, préalablement défini en réalisant une réaction électrochimique de l'enzyme seule, en faisant varier le potentiel de l'électrode, en mesurant la vitesse de transformation de l'enzyme et en détectant le potentiel pour lequel cette vitesse présente une valeur maximale ou proche de la maximale.

3. Procédé de mesure selon la revendication 2, CARACTERISE EN CE QUE la définition du potentiel de régénération est réalisée

en mesurant l'intensité du courant produit dans la réaction électrochimique de l'enzyme seule, cette intensité étant proportionnelle à la vitesse de transformation de l'enzyme.

4. Procédé de mesure selon la revendication 1 ou 2, CARACTERISE EN CE QU'il consiste à disposer au voisinage de la chambre réactionnelle une électrode de référence pour permettre de fixer le potentiel de l'électrode de régénération enzymatique à une valeur sensiblement constante appropriée.

5. Procédé de mesure selon la revendication 1, CARACTERISE EN CE QUE l'enzyme est préalablement adsorbée sur l'électrode de régénération avant immersion dans ladite chambre dans la solution de substrat.

6. Procédé de mesure dans lequel le substrat oxydable est du lactate, CARACTERISE EN CE QUE l'enzyme spécifique utilisée est une lacticodeshydrogénase sous forme oxydée.

7. Procédé de mesure selon les revendications 4 et 6, CARACTERISE EN CE QUE la différence de potentiel entre l'anode de régénération enzymatique et l'électrode de référence est sensiblement fixée à une valeur comprise entre 450 et 550 millivolts en supposant l'électrode de référence au calomel saturé.

8. Procédé de mesure dans lequel le substrat oxydable est du glucose, CARACTERISE EN CE QUE l'enzyme spécifique utilisée est une glucose oxydase sous forme oxydée.

9. Procédé de mesure selon les revendications 4 et 8, CARACTERISE EN CE QUE la différence de potentiel entre l'anode de régénération enzymatique et l'électrode de référence est sensiblement fixée à une valeur comprise entre 400 et 550 millivolts en supposant l'électrode de référence au calomel saturé.

10. Procédé de mesure de la concentration d'un substrat de masse moléculaire très inférieure à celle de l'enzyme spécifique, CARACTERISE EN CE QUE l'on utilise une membrane semi-perméable ayant une porosité définie par un seuil de coupure de valeur intermédiaire entre la masse moléculaire du substrat et celle de l'enzyme.

11. Procédé de mesure de la concentration de lactate ou de glucose dans une solution selon la revendication 6 ou 8, CARACTERISE EN CE QUE la concentration de l'enzyme spécifique lacticodeshydrogénase ou glucose oxydase, disposée dans la chambre réactionnelle est approximativement comprise entre 80 et 500 micromoles par litre.

12. Dispositif de mesure de la concentration d'un substrat en solution permettant la mise en oeuvre du procédé conforme à l'une de revendication 1 à 11 comprenant un corps délimitant une chambre réactionnelle, une membrane semi-perméable fixée sur ce corps et fermant ladite chambre, une anode située dans cette chambre, une cathode à l'extérieur de celle-ci et une solution enzymatique contenue dans la chambre, CARACTERISE EN CE QUE ladite chambre contient en solution une enzyme spécifique du substrat à l'exclusion de tout cofacteur actif.

13. Dispositif de mesure selon la revendication 12, CARACTERISE EN CE QUE l'enzyme se trouve initialement adsorbée sur l'anode disposée dans ladite chambre .

14. Dispositif de mesure selon la revendication 12, CARACTERISE EN CE QUE la cathode disposée à l'extérieur de ladite chambre est une électrode insoluble dans la solution de substrat et inattaquable.

15. Dispositif de mesure selon la revendication 12, CARACTERISE EN CE QUE la membrane semi-perméable présente une porosité définie par un seuil de coupure de l'ordre de 4000 à 6000.

16. Dispositif de mesure selon la revendication 12, CARACTERISE EN CE QUE ledit corps est cylindrique et est pourvu en bout d'une partie creuse fermée par la membrane semi-perméable, l'anode étant assujettie en bout dudit corps dans ladite partie creuse et la cathode de forme annulaire étant assujettie autour dudit corps au voisinage de son extrémité.

17. Dispositif de mesure selon la revendication 12, CARACTERISE EN CE QU'il comprend une électrode de référence prolongée jusqu'au voisinage de la chambre réactionnelle par une jonction électrolytique.

18. Dispositif de mesure selon la revendication 12, CARACTERISE EN CE QUE L'électrode de régénération enzymatique est réalisée en un métal ou alliage non oxydable au potentiel de travail.

19. Dispositif de mesure selon la revendication 18, CARACTERISE EN CE QUE l'électrode de régénération enzymatique est choisie en un métal du groupe suivant : platine, or, rhodium, irridium ou en un alliage desdits métaux.

20. Dispositif de mesure selon la revendication 12, CARACTERISE EN CE QUE l'électrode de régénération enzymatique est réalisée en carbone vitreux.

21. Dispositif de mesure selon la revendication 12, CARACTERISE EN CE QUE l'électrode de régénération enzymatique est réalisée en un métal recouvert d'une couche d'oxyde conducteur électronique.

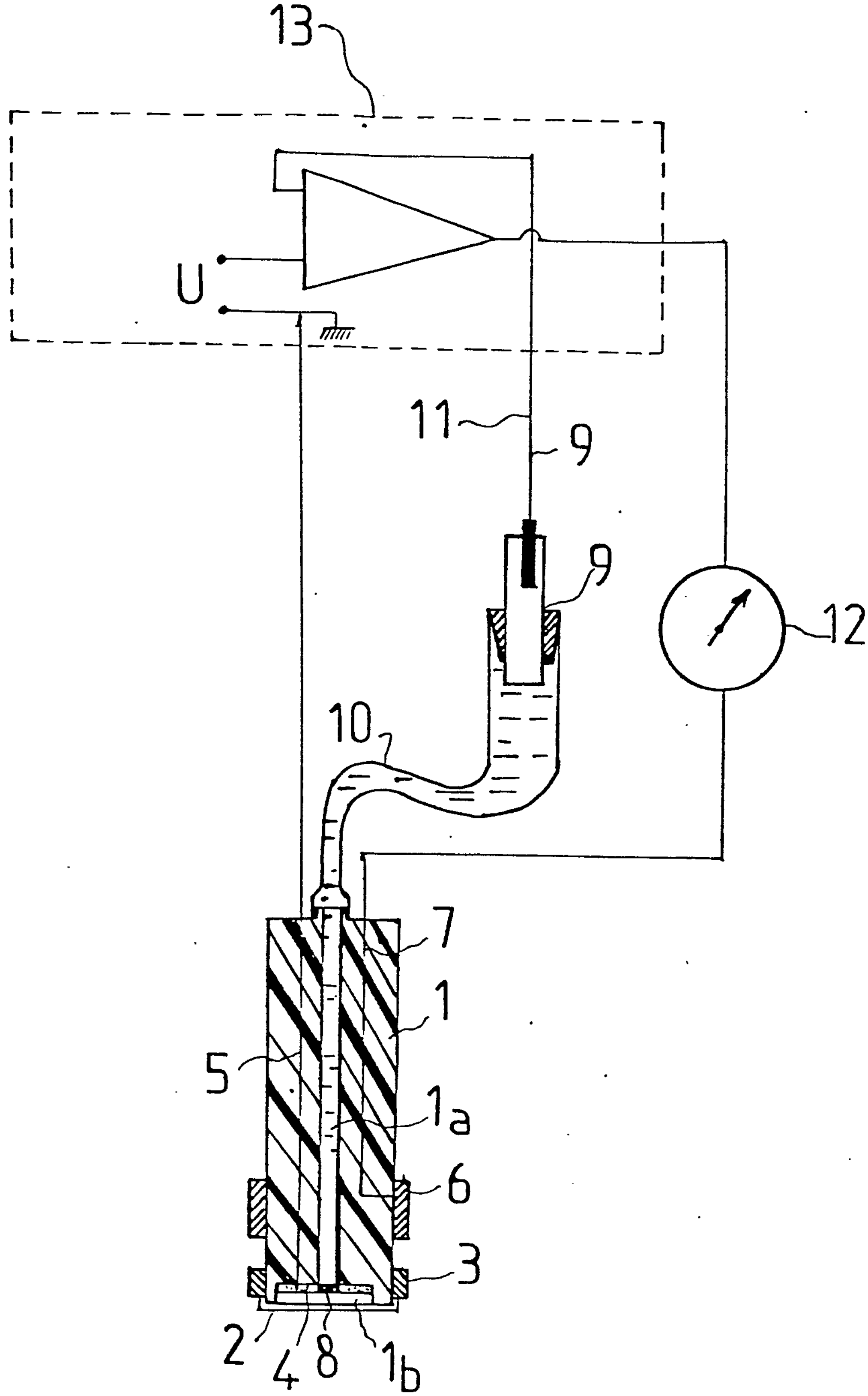


Fig.1

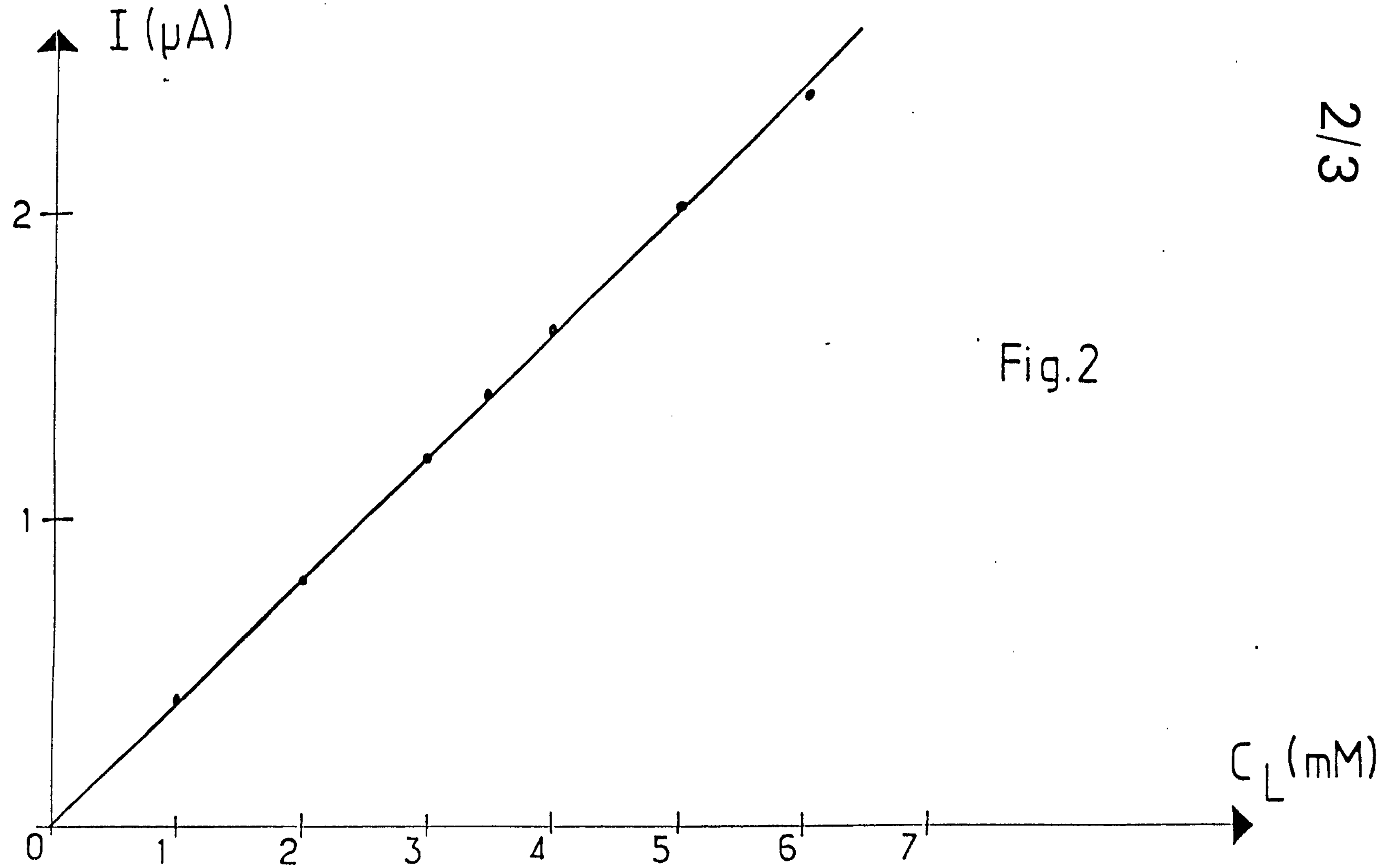


Fig.2

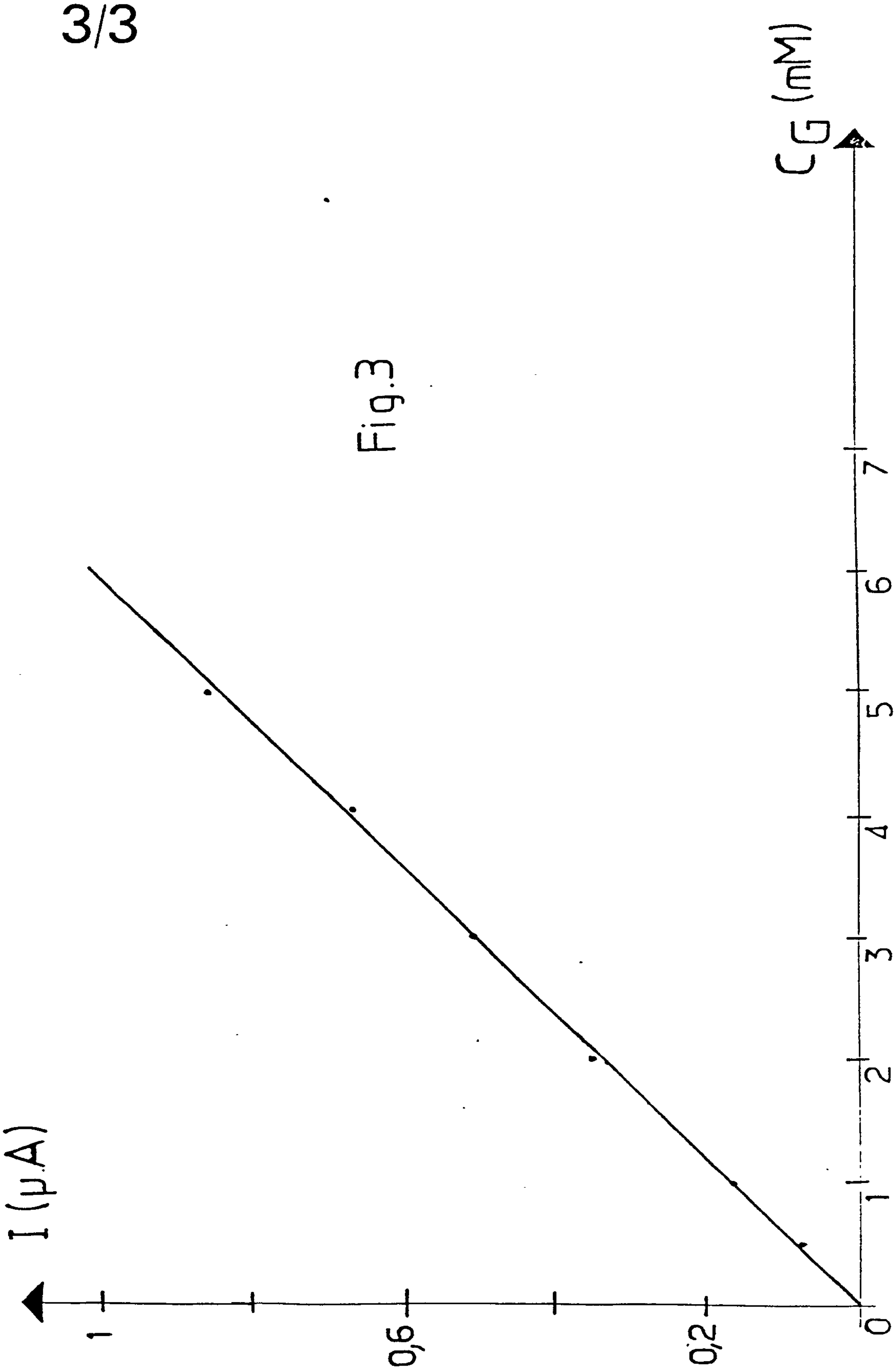


Fig.3

0030195

Office européen des brevets

# RAPPORT DE RECHERCHE EUROPEENNE

Numéro de la demande

EP 80 40 1711

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| | CHEMICAL ABSTRACTS, vol. 88, 13-02-1978, no. 7, page 214, réf.: 46811k<br>Columbus, Ohio, US<br>S.D. VARFOLOMEEV "Bioelectrocatalysis. Hydrogenase as catalyst of electrochemical hydrogen ionization"<br>& Bioelectrochem. Bioenerg. 1977, 4(3), 314-26<br>* Abrégé en entier *<br>-- | 1,2,6 | C 12 Q 1/00<br>1/32<br>1/54<br>G 01 N 27/30 |
| | | | **DOMAINES TECHNIQUES RECHERCHES (Int. Cl. 3)** |
| | CHEMICAL ABSTRACTS, vol. 83, 07-07-1975, no. 1, page 159, réf.: 1614d<br>Columbus, Ohio, US<br>M. NANJO et al.: "Amperometric determination of alcohols, aldehydes and carboxylic acids with an immobilized alcohol oxidase enzyme electrode"<br>& Anal. Chim. Acta 1975, 75(1), 169-80.<br>* Abrégé en entier *<br>-- | 1 | C 12 Q 1/00<br>1/26<br>1/32<br>1/54<br>C 12 M 1/40<br>G 01 N 27/30 |
| | IBM TECHNICAL DISCLOSURE BULLETIN vol. 11, no. 4, septembre 1968<br>New York US<br>G.W. NEFF et al.: "Direct analysis of urea in biological fluids", pages 374, 375<br>* Document en entier *<br>-- | 1,12-19 | **CATEGORIE DES DOCUMENTS CITES**<br>X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons |
| | DE - A - 2 612 433 (SERVO CHEM. A.B.)<br>* Page 6, revendications 1-3; page 9, figure 1 *<br>-- ./. | 12,13 | &: membre de la même famille, document correspondant |

X Le présent rapport de recherche a été établi pour toutes les revendications

| Lieu de la recherche | Date d'achèvement de la recherche | Examinateur |
|---|---|---|
| La Haye | 28-02-1981 | WALLINDER |

0030195

Office européen des brevets

**RAPPORT DE RECHERCHE EUROPEENNE**

Numéro de la demande

EP 80 40 1711

-2-

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| A | DE - A - 2 659 071 (WISCONSIN ALUMINI RESEARCH FOND)<br>* Pages 1-3; revendications 1-12; page 18, ligne 17 - page 19, ligne 4; figure 1 *<br>-- | 1,6, 12 | |
| A | FR - A - 2 152 219 (F. HOFFMANN-LA ROCHE)<br>* Pages 8,9; revendications 1-12 *<br>-- | 1,12 | DOMAINES TECHNIQUES RECHERCHES (Int. Cl. 3) |
| E | WO - A - 80/00453 (NATIONAL RESEARCH DEVELOPMENT CORP.)<br>* Abrégé; page 17, revendications 1-4 *<br>-- | 1,2,6 | |
| E | WO - A - 80/00850 (SETRIC)<br>* Abrégé; pages 11-13, revendications 1-12; figures 1 et 2 *<br>& FR - A - 2 439 995<br>---- | 1,12, 15-19 | |

OEB Form 1503.2 06.78